# NEUE EUROPÄISCHE PATENTSCHRIFT

(11) **EP 0 777 486 B2**
(45) Veröffentlichungstag und Bekanntmachung des Hinweises auf die Entscheidung über den Einspruch: **13.06.2007**
(45) Hinweis auf die Patenterteilung: 16.04.2003
(21) Anmeldenummer: 95931192.9
(22) Anmeldetag: 23.08.1995
(51) Int. Cl.: A61K 31/70, A23L 1/30

(54) **ALLERGIEPROTEKTIVE FORMELNAHRUNG MIT GANGLIOSIDEN**
ALLERGY-PROTECTIVE FORMULA FOOD WITH GANGLIOSIDES
ALIMENTS DE FORMULE SPECIFIQUE PROTEGEANT CONTRE LES ALLERGIES ET CONTENANT DES GANGLIOSIDES

(30) Priorität: 24.08.1994 DE 4430041
(43) Veröffentlichungstag der Anmeldung: 11.06.1997
(73) Patentinhaber: N.V. Nutricia, 2712 HM Zoetermeer (NL)
(72) Erfinder: SCHROTEN, Horst, D-40764 Langenfeld (DE)
(74) Vertreter: Köster, Hajo
(86) Internationale Anmeldenummer: PCT/EP1995/003346
(87) Internationale Veröffentlichungsnummer: WO 1996/005844

(56) Entgegenhaltungen:
- EP-A- 0 251 298
- WO-A-92/17189
- LIFE SCI., Bd. 51, Nr. 11, 1992 Seiten 847-851, T. IKEDA ET AL. 'ganglioside-induced inhibition of in-vivo immune response in mice.'
- ANN. NEUROL., Bd. 27, Nr. spl., 1990 Seiten S69-S74, R.W. LEDEEN ET AL. 'Gangliosides offer partial protection in experimental allergic neuritis.'
- CELL. IMMUNOL., Bd. 154, Nr. 1, 1994 Seiten 231-239, K. SHIMADA ET AL. 'Suppression of experimental allergic encephalomyelitis in Lewis rats by administration of gangliosides.'
- DATABASE WPI Week 9347 Derwent Publications Ltd., London, GB; AN 93-360071 & JP,A,05 276 894 (SNOW BRAND MILK PROD CO LTD) , 26.Oktober 1993
- DATABASE WPI Week 9346 Derwent Publications Ltd., London, GB; AN 93-364427 & JP,A,05 269 353 (SNOW BRAND MILK PROD CO LTD) , 19.Oktober 1993
- Abstract JP 06-016536
- Guesry, M.D. et al (1991) "Milk Formulae in the Prevention of Food Allergy"; Allergy Proc., vol. 12(4):221-226
- Hurrell, R. et al (1989) "Micronutrients in milk and milk-based food products"; Elsevier Science Publishers, England, pp. 262-287
- Jensen C. et al (1987) "Complexity of the influence of gangliosides on histamine release from human basophils and rat mast cells"; Agents Actions, vol. 21, pp. 79-82
- Carlson S. et al (1985) "N-Acetylneuraminic acid concentrations in human milk olifosaccharides and glycoproteins during lactation"; Am. J. Clin. Nutr., vol. 41, pp. 720-726
- Abstract JP 05-276894, 26 October 1993
- Helm, R.M. et al (Dec 2000) "Mechasnism of food allergy"; Curr Opin Ummunol. 12(6):647-653 (abstract)
- Rueda, R. et al (Dec 1998) "Neonatal dietary gangliosides"; Early Hum. Dev. 53 Suppl., pp. 135-147 (abstract)
- Boehm, G. et al (Jul 2004) "Prebiotics in infant formulas"; J Clin Gastroenterol., 38 (6 Suppl):S76-9 (abstract)
- Vazques, E. et al (2001) "Dietary gangliosides positively modulate the percentages of Th1 and Th2 lymphocyte subsets in small intestine of mice at weaning"; Biofactors, 15(1):1-9 (abstract)

## Beschreibung

Es ist schon seit längerer Zeit bekannt, daß gestillte Kinder weniger häufig an Allergien erkranken als nicht gestillte Kinder. Für dieses Phenomen werden verschiedene Ursachen diskutiert. So sollen beispielsweise im Kolostrum Substanzen vorhanden sein, die die Reifung der Darmschleimhaut beschleunigen. Auch soll die Armut der Muttermilch an Fremdeiweißen dafür verantwortlich sein. Zudem sollen eine Vielzahl antiinfektiöser und antiinflammatorischer Faktoren ursächlich sein, welche die negative Wirkung bakterieller Toxine auf die Darmschleimhaut verhindern. Die genauen wissenschaftlichen Zusammenhänge sind jedoch noch nicht bekannt.

Muttermilch enthält neben zahlreichen anderen Bestandteilen auch verschiedene Ganglioside. Die Hauptquelle der Ganglioside in der menschlichen Milch sind die Milchfettkügelchenmembranen. Der Anteil der verschiedenen Ganglioside in der menschlichen Milch ist lactationsphasenabhängig. Im Kolostrum dominiert das Gangliosid GD3 (Verhältnis GM3/GD3: 0,2 - 0,3). In der späteren Phase (60. - 360. Tag) dominiert das Gangliosid GM3 (Verhältnis GM3/GD3: > 3).

Ganglioside sind Lipide, die N-Acetylneuraminsäure enthalten. Sie sind integraler Bestandteil von Zellmembranen und spielen eine wichtige Rolle bei Zellerkennungsmechanismen (beispielsweise Bindung von Toxinen) und anderen elementaren physiologischen Vorgängen. So sind die Ganglioside mitverantwortlich für die Proliferation und Differenzierung sowie beim Krebsartigwerden von Zellen.

Bis data was bekannt, dass die intramuskuläre Applikation von Gangliosiden allergische Reaktionen bei Mausen unterdrückt (Life Sci., Bd.51, Nr. 11, 1992 T.IKEDA ET AL. ganglioside induced inhibition of in - vivo immune response in mice).

Aufgabe der vorliegenden Erfindung ist es, einen verbesserten Schutz vor Allergien zu ermöglichen.

Gelöst wird diese Aufgabe durch die Lehre des Anspruches 1.

N-acetyl-neuraminsäurehaltige Ganglioside können die Histaminfreisetzung aus menschlichen basophilen Granulozyten hemmen. Es wird angenommen, daß die immunmodulatorischen Eigenschaften von Gangliosiden in der frühen Phase der Allergieentstehung (Antigenerkennung) eine Rolle spielen, während die Histaminfreisetzung zu den Endgliedern der allergischen Reaktionskette zählt.

Es wurde nun überraschend gefunden, daß N-acetyl-neuraminsäurehaltige Ganglioside über eine allergieprotektive Wirkung verfügen. Daher werden derartige Ganglioside erfindungsgemäß einer Formelnahrung zugegeben.

Unter einer Allergieprotektion wird im Rahmen der vorliegenden Unterlagen nicht nur eine Verhinderung des erstmaligen Auftretens von Allergien und somit eine Prävention, sondern auch ein Schutz bei einer bereits manifesten Allergie vor einem erneuten Auftreten dieser Allergie verstanden.

Unter einer Formelnahrung wird im Rahmen der vorliegenden Erfindung jedwede auf Basis von tierischen und/oder pflanzlichen Ursprungsstoffen hergestellte künstliche Nahrung verstanden. Dazu zählen insbesondere Säuglingsnahrungen, Säuglingsmilchnahrungen und Frühgeborenennahrungen.

Derartige Formelnahrungen enthalten üblicherweise Proteine, Kohlenhydrate und/oder Fette als Hauptbestandteile. Diese Formelnahrungen können in fester oder flüssiger Form vorliegen und entweder direkt oder nach Zugabe von Milch und/oder Wasser oral verabreicht werden. Derartige Formelnahrungen sind per se bekannt, so daß sich eine weitere Erörterung erübrigt.

Die Ganglioside werden den Formelnahrungen für Frühgeborene, Säuglinge und Kleinkinder von Menschen vorzugsweise in einer solchen Menge einverleibt, daß diese Ganglioside vorzugsweise 0,1 bis 70 mg, weiterhin bevorzugt 1 bis 35 mg und insbesondere 1 bis 10 mg pro Liter der fertigen, flüssigen Nahrung ausmachen. Stellt eine derartige Formelnahrung beispielsweise ein pulvriges Produkt dar, das noch in Milch und/oder Wasser aufzulösen ist, dann werden die Ganglioside dieser pulvrigen Formelnahrung in einer solchen Menge zugegeben, daß die Ganglioside nach Zugabe der bestimmungsgemäßen Menge an Milch und/oder Wasser in den oben angeführten Mengen in der fertigen, flüssigen und somit einem Kind zu verabreichenden Nahrung vorliegen. Feste Formelnahrungen, die noch in Milch und/oder Wasser aufzulösen sind, enthalten daher vorzugsweise 0,7 bis 400 mg, insbesondere 7 bis 245 mg und weiter insbesondere 7 bis 70 mg an Gangliosiden pro kg der festen Formelnahrung.

Bei den obigen Berechnungen wurde davon ausgegangen, daß ein humaner Säugling etwa 1 l flüssiger Nahrung pro Tag zu sich nimmt und ein Gewicht von ca. 4 kg besitzt.

Die hier in Rede stehenden Ganglioside können erfindungsgemäß nicht nur Säuglingen und Kleinkindern von Menschen, sondern auch jugendlichen und erwachsenen Menschen verabreicht werden. Die Ganglioside werden dazu vorzugsweise in einer Menge von 0,625 mg bis 440 mg, insbesondere von 6,25 mg bis 220 mg und weiter insbesondere von 6,25 mg bis 63 mg pro Tag an einen erwachsenen Menschen oder an einen jugendlichen Menschen verabreicht.

Als N-acetyl-neuraminsäurehaltiges Gangliosid werden vorzugsweise die Ganglioside GM3, GD3 oder GT1b alleine oder in beliebiger Mischung aus einem oder mehreren dieser Ganglioside eingesetzt.

Als Quellen für die erfindungsgemäß eingesetzten Ganglioside können die Milchen von Säugetieren, beispielsweise von Kuh, Schaf, Ziege, Pferd, Schwein oder Kamel, dienen. Es ist auch möglich, die entsprechenden gangliosid-reichen Gewebe, beispielsweise Gehirn, von den genannten Tieren als Ausgangsprodukt einzusetzen. Als Ausgangsprodukt kommen ferner Weizenproteinkonzentrat und insbesondere Buttermilch in Frage.

Die Ganglioside bzw. die Gangliosidzusammensetzung kann in großen Mengen aus Milch nach bekannten Techniken gewonnen werden. Diesbezüglich wird beispielsweise auf die JP-A 269992/1988 verwiesen.

Sofern man das Verhältnis von dem Gangliosid GD3, das in derartigen Ausgangsprodukten vorwiegend vorhanden ist, zu dem Gangliosid GM3 verändern möchte, ist es möglich, eine derartige GD3-Zusammensetzung oder auch Ausgangsprodukte, wie Kuhmilch, Buttermilch und Weizenkonzentrat, direkt zu behandeln, beispielsweise mit Chlorwasserstoffsäure, Milchsäure oder Zitronensäure, so daß ein Sialinsäuremolekül vom Gangliosid GD3 hydrolysiert wird, wodurch GM3 hergestellt wird. Ferner ist es möglich, die genannten Ausgangsprodukte direkt mit Sialidase oder einer Säure zu behandeln. Derartige Herstellungsweisen sind beschrieben in AU-A-32004/93.

Es können im übrigen auch im Handel erhältliche Ganglioside und insbesondere die Ganglioside GM3, GD3 und GT1b eingesetzt werden. Derartige Ganglioside werden von der Firma Sigma angeboten. Es muß natürlich sichergestellt werden, daß die im Handel erhältlichen Ganglioside unter Beachtung der einschlägigen lebensmittelrechtlichen Vorschriften hergestellt wurden.

Ferner ist es möglich, aus Tiergehirnen gemäß dem in EU-A 0 365 861 beschriebenen Verfahren Glykolipide herzustellen und daraus durch Abspaltung von Neuraminsäure, beispielsweise nach JP-A-269992, oder säulenchromatographisch (DEAE Sephadex A25) Ganglioside zu gewinnen.

Die unter Einsatz von Gangliosiden herzustellende Säuglingsnahrung liegt üblicherweise in Form eines sofort löslichen Pulvers oder einer Flüssigmilch vor. Das flüssige Produkt wird üblicherweise sterilisiert oder bei Ultrahochtemperatur sterilisiert und aseptisch abgefüllt.

Die Substanzen, die dabei eingesetzt werden können, sind beispielsweise folgende:
a) demineralisiertes Molkenpulver
b) Milchzucker
c) Magermilch flüssig (8,5 %)
d) Mineralsalze
e) Vitamine
f) Fettmischung
g) erfindungsgemäß eingesetzte Ganglioside bzw. Gangliosid-Mischung.

Die Herstellung einer derartigen Säuglingsnahrung ist beispielsweise beschrieben in DE-A-39 20 679. Auf die entsprechende Offenbarung wird hiermit ausdrücklich Bezug genommen.

Die Ganglioside bzw. die Gangliosid-Mischung wird zusammen mit den übrigen üblichen Bestandteilen verarbeitet.

Eine vorteilhafte erfindungsgemäße Gangliosid-Mischung ist beispielhaft wie folgt aufgebaut:
- GM3 :: 1 mg
- GD3 :: 30 mg
- GTb1 :: 15 mg.

## Patentansprüche

1. Verwendung von N-acetylneuraminsäure-haltigen Gangliosiden zur Herstellung von Formelnahrungen zur Allergieprotektion beim Menschen.

2. Verwendung von N-acetylneuraminsäure-haltigen Gangliosiden nach Anspruch 1 zur Allergieprotektion bei Frühgeborenen, Säuglingen und Kleinkindern.

3. Verwendung von N-acetylneuraminsäure-haltigen Gangliosiden nach Anspruch 2 in einer flüssigen Nahrung in einer Menge von 0,1 bis 70 mg, insbesondere 1 bis 35 mg, weiterhin insbesondere von 1 bis 10 mg pro Liter der fertigen, flüssigen Nahrung.

4. Verwendung von N-acetylneuraminsäure-haltigen Gangliosiden nach Anspruch 2 in einer festen Nahrung in einer Menge von 0,7 bis 400 mg, insbesondere von 7 bis 245 mg, sowie weiter insbesondere von 7 bis 70 mg, pro kg der festen Nahrung.

5. Verwendung von N-acetylneuraminsäure-haltigen Gangliosiden nach Anspruch 1 bei erwachsenen und jugendlichen Menschen in einer Menge von 0,625 mg bis 440 mg, insbesondere von 6,25 mg bis 220 mg und weiter insbesondere von 6,25 mg bis 63 mg pro Tag.

6. Verwendung nach einem der Ansprüche 1 bis 5,
**dadurch gekennzeichnet,**
**dass** die N-acetylneuraminsäure-haltigen Ganglioside GM3, GD3 oder GT1b oder eine Mischung davon sind.

## Claims

1. Use of N-acetylneuraminic acid-containing gangliosides for the production of formula foods for allergy in humans.

2. Use of the N-acetylneuraminic acid-containing gangliosides according to Claim 1 for allergy protection in premature and newborn babies and in infants.

3. Use of the N-acetylneuraminic acid-containing gangliosides according to Claim 2 in a liquid food in an amount of 0.1 to 70 mg, particularly 1 to 35 mg, more particularly from 1 to 10 mg per litre of the finished, liquid food.

4. Use of the N-acetylneuraminic acid-containing gangliosides according to Claim 2 in a solid food in an amount of 0.7 to 400 mg, particularly 7 to 245 mg, and more particularly from to 70 mg, per kg of the solid food.

5. Use of the N-acetylneuraminic acid-containing gangliosides according to Claim 1 in adult and adolescent humans in am amount of 0.625 mg to 440 mg, particularly 6.25 mg to 220 mg and more particularly from 6.25 mg to 63 mg per day.

6. Use according to one of Claims 1 to 5, **characterised in that** the N-acetylneuraminic acid-containing gangliosides are GM3, GD3 or GT1b or a mixture thereof.

## Revendications

1. Mise en oeuvre de gangliosides contenant de l'acide N-acétylneuraminique pour la fabrication d'aliments artificiels de protection contre les allergies chez l'Homme.

2. Mise en oeuvre des gangliosides contenant de l'acide N-acétylneuraminique selon la revendication 1 pour une protection contre les allergies chez les nouveau-nés, les nourrissons et les enfants en bas âge.

3. Mise en oeuvre des gangliosides contenant de l'acide N-acétylneuraminique selon la revendication 2 dans un aliment liquide, à raison de 0,1 à 70 mg, en particulier de 1 à 35 mg, de façon plus particulièrement préférée, de 1 à 10 mg par litre d'aliment liquide final.

4. Mise en oeuvre des gangliosides contenant de l'acide N-acétylneuraminique selon la revendication 2 dans un aliment solide, à raison de 0,7 à 400 mg, en particulier de 7 à 245 mg, et de façon plus particulièrement préférée, de 7 à 70 mg, par kg d'aliment solide.

5. Mise en oeuvre des gangliosides contenant de l'acide N-acétylneuraminique selon la revendication 1 chez les adultes et les adolescents, à raison de 0,625 mg à 440 mg, en particulier de 6,25 mg à 220 mg, et de façon plus particulièrement préférée, de 6,25 mg à 63 mg par jour.

6. Mise en oeuvre selon l'une quelconque des revendications 1 à 5, **caractérisée en ce que** les gangliosides contenant de l'acide N-acétylneuraminique sont les gangliosides GM3, GD3 ou GT1b, ou un mélange de ceux-ci.
